# EUROPEAN PATENT SPECIFICATION

(11) **EP 3 656 357 B1**
(45) Date of publication and mention of the grant of the patent: **11.08.2021**
(21) Application number: 17784329.9
(22) Date of filing: 20.07.2017
(51) Int. Cl.: A61F 2/30

(54) **TEMPOROMANDIBULAR ARTICULATION REPLACEMENT SYSTEM**
KIEFERGELENK ERSATZ SYSTEM
SYSTÈME DE REMPLACEMENT D'ARTICULATION TEMPOROMANDIBULAIRE

(43) Date of publication of application: 27.05.2020
(73) Proprietor: Roselló Oyaga, Javier, 28660 Boadilla del Monte (Madrid) (ES); Ramos Rodriguez, Laura, 28660 Boadilla del Monte (Madrid) (ES)
(72) Inventor: Roselló Oyaga, Javier, 28660 Boadilla del Monte (Madrid) (ES); Ramos Rodriguez, Laura, 28660 Boadilla del Monte (Madrid) (ES)
(74) Representative: Pons
(86) International application number: PCT/ES2017/070525
(87) International publication number: WO 2019/016415

(56) References cited:
- EP-A1- 2 810 621
- WO-A1-2014/043452
- CN-A- 104 887 352
- US-A- 5 549 680
- US-A- 6 132 466

## Description

### OBJECT OF THE INVENTION

The present invention falls within the technical field of temporomandibular joint prostheses and relates to a replacement system for said temporomandibular joint comprising at least one prosthesis configured to be implanted in the mandible to functionally reconstruct a diseased and/or damaged joint.

The temporomandibular joint replacement system of the present invention allows replacing the piece of the prosthesis subjected to wear in an easy and minimally invasive way for the patient.

### BACKGROUND OF THE INVENTION

Temporomandibular joint (TMJ) prostheses that are known in the state of the art are implanted in a patient under the following indications:
- Arthritic conditions: osteoarthritis, traumatic arthritis, and rheumatoid arthritis.
- Ankylosis, including, but not limited to, recurrent ankylosis with excessive heterotopic ossification.
- Revision procedures in which other treatments (for example, alloplastic reconstruction or autologous grafts) have failed.
- Avascular necrosis.
- Severe fractures.
- Functional deformities.
- Repeatedly intervened joints.
- Neoplasms or malignancy (for example, tumor excision).
- Developmental abnormalities.
- Degenerated or reabsorbed joints with severe anatomical discrepancies.
- Cases in which the surgeon considers it necessary.

Temporomandibular joint (TMJ) prostheses are made up of 2 components, a mandibular component (ramus and condyle) and a cranial component (fossa). The mandibular component is usually manufactured with a chromium-cobalt-molybdenum alloy in the ramus and titanium in the condylar head. The fossa component is usually manufactured with ultra-high-molecular-weight polyethylene (UHMWPE) on the joint contact surface with the condyle head, and with a titanium mesh or a titanium plate for the surfaces in contact with the bone of the base of the cranium and the zygomatic arch.

Some versions require very large mandibular components and house large screws in order to ensure the stability thereof over time.

The use of large amounts of materials other than titanium increases the possibilities of rejection by the patient, since titanium is currently the most biocompatible material.

The use of polyethylene components in their entirety makes it difficult to diagnose an eventual problem, because it is radiolucent, that is, it is not visible in X-rays and in computed tomographies (CT).

In the case of polyethylene wear, it is necessary to change the cranial component.

Document WO2014/043452A1 discloses a temporomandibular joint replacement system according to the preamble of claim 1. The temporomandibular joint replacement system of the present invention solves all of the aforementioned drawbacks.

### DESCRIPTION OF THE INVENTION

The present invention solves the technical problem indicated above by means of a temporomandibular joint replacement system, which allows replacing the piece of the prosthesis subjected to wear in an easy and minimally invasive way for the patient.

The temporomandibular joint replacement system comprises at least one prosthesis, which in turn comprises:
- a mandibular component, and
- a cranial component comprising a first piece, which is arranged in contact with the mandibular component,
wherein the first piece of the cranial component is detachable from the cranial component when the prosthesis is implanted in the patient.

In this way, the first piece of the cranial component can be changed when it has been worn out, and in a minimally invasive manner for the patient, since it is not necessary to change the entire cranial component, as in the state of the art, wherein the first piece of the cranial component cannot be detached from the cranial component when the prosthesis is arranged in the patient. The only way to separate the first piece from the cranial component would be by forcing the separation thereof, which does not fall within the definition of a detachable piece. Furthermore, this could only be accomplished by removing the entire cranial component from the patient, since attempting to separate the first piece with the cranial component implanted in the patient is not feasible due to the damage such action could cause to the patient, in addition to future problems associated with misalignments of at least the cranial component.

The temporomandibular joint replacement system thus constituted allows a correct placement of the new center of rotation of the mandibular component, approximately 12-15 mm below the cranial component and with an advance of 4-5 mm in the frontal direction. This allows the possibility of displacement in both the anterior-posterior and medial-lateral directions of the mandibular component with respect to the cranial component of approximately 3 to 4 mm.

Optionally, the cranial component comprises a second piece configured to be anchored to the zygomatic arch of the patient, wherein preferably the first piece of the cranial component is detachable from the second piece of the cranial component when the prosthesis is implanted in the patient.

Optionally, the cranial component comprises guiding means configured to enable detaching the first piece of the cranial component with respect to said cranial component when the prosthesis is implanted in the patient. Preferably, the first piece of the cranial component comprises first guiding means and the second piece of the cranial component comprises second guiding means, wherein the first guiding means contribute with the second guiding means to enable detaching the first piece of the cranial component with respect to the second piece of the cranial component when the prosthesis is implanted in the patient.

Optionally, the cranial component comprises locking means, which prevent relative movement between the first piece of the cranial component and the cranial component when the prosthesis is implanted in the patient. Preferably, the second piece of the cranial component comprises locking means, which prevent relative movement between the first piece of the cranial component and the second piece of the cranial component when the prosthesis is implanted in the patient.

The mandibular component comprises a first piece arranged in contact with the first piece of the cranial component, wherein the first piece of the mandibular component is exchangeable. Preferably, the first piece of the mandibular component is spherical.

Moreover, the mandibular component comprises a second piece, which is attachable to the mandible of the patient, preferably attachable by means of screws to the mandible of the patient.

The first piece of the mandibular component is suitable to be positioned at different heights with respect to the second piece of the mandibular component.

Optionally, the temporomandibular joint replacement system further comprises auxiliary elements configured to carry out the implantation of the prosthesis in the patient.

Preferably, the auxiliary elements comprise at least one cutting guide and/or at least one cutting indicator and/or at least one spacing indicator.

### DESCRIPTION OF THE DRAWINGS

In order to implement the present description and for the purpose of helping to make the characteristics of the invention more readily understandable according to a preferred embodiment thereof, this description is accompanied by a set of drawings, which, by way of illustration and not limitation, represent the following:
Figure 1 shows a perspective view of the temporomandibular joint replacement system of the present invention.
Figure 2 shows a perspective view of the temporomandibular joint replacement system of the present invention implanted in a patient.
Figure 3 shows a perspective view of the first piece of the cranial component when it is attached to the second piece of the cranial component of the temporomandibular joint replacement system of the present invention, with the prosthesis implanted in the patient.
Figure 4 shows a perspective view of the first piece of the cranial component when it is partially detached from the second piece of the cranial component (not shown in its entirety) when the prosthesis is implanted in the patient.
Figure 5 shows a perspective view of Figure 3, wherein the locking means of the second piece of the cranial component are shown, which prevent relative movement between the first piece of the cranial component and the second piece of the cranial component when the prosthesis is implanted in the patient.
Figure 6 shows an elevation view of the first piece of the mandibular component positioned at different heights with respect to the second piece of the mandibular component.
Figure 7 shows an elevation view of the cutting guides and spacing indicator of the temporomandibular joint replacement system of the present invention implanted in a patient.
Figure 8 shows an elevation view of the cutting guides of the temporomandibular joint replacement system of the present invention implanted in a patient prior to performing the osteotomies.
Figure 9 shows an elevation view of the cutting guides of the temporomandibular joint replacement system of the present invention implanted in a patient after performing the osteotomies.
Figure 10 shows a perspective view of the cranial cutting indicators implanted in a patient after performing the osteotomies.
Figure 11 shows a perspective view of the mandibular cutting indicator implanted in a patient after performing the osteotomies.
Figure 12 shows a second embodiment of the temporomandibular joint replacement system of the present invention implanted in a patient wherein the mandibular component replaces the mandible in its entirety.

### PREFERRED EMBODIMENT OF THE INVENTION

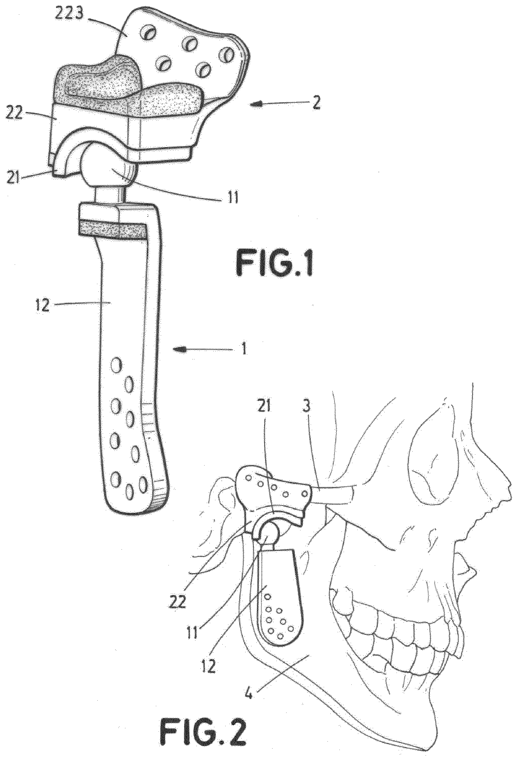

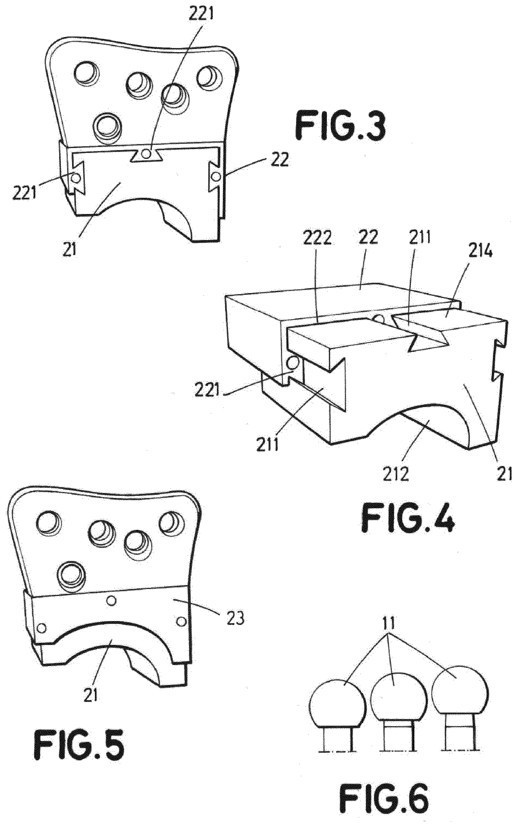

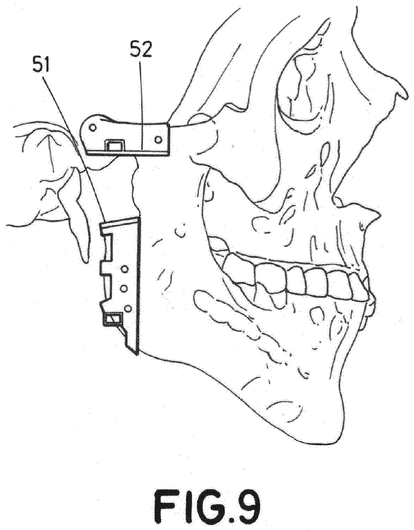

A detailed description of a preferred embodiment of the present invention is described below, in accordance with Figures 1 to 12 mentioned above.

The temporomandibular joint replacement system comprises at least one prosthesis, which in turn comprises:
- a mandibular component (1), and
- a cranial component (2) comprising a first piece (21), which is arranged in contact with the mandibular component (1),
wherein the first piece (21) of the cranial component (2) is detachable from the cranial component (2) when the prosthesis is implanted in the patient.

In this preferred embodiment, the cranial component (2) comprises a second piece (22) configured to be anchored to the zygomatic arch (3) of the patient by means of a lateral surface (223) of the second piece (22) of the cranial component (2), wherein the first piece (21) of the cranial component (2) is detachable from the second piece (22) of the cranial component (2) when the prosthesis is implanted in the patient. The lateral surface (223) of the second piece (22) of the cranial component (2) comprises holes for anchoring by screwing the second piece (22) of the cranial component (2) to the zygomatic arch (3) of the patient.

The first piece (21) of the cranial component (2) comprises a lower surface (212), which is arranged in contact with the mandibular component (1), wherein said lower surface (212) is preferably cylindrical or frustoconical. The lower surface (212), which is arranged in contact with the mandibular component (1), comprises a semi-spherical housing (213), preferably with a diameter of 8 mm.

The first piece (21) of the cranial component (2) comprises an upper surface (214) and the second piece (22) of the cranial component (2) comprises an inner surface (222), wherein the upper surface (212) of the first piece (21) of the cranial component (2) is configured to slide along the inner surface (222) of said lower surface (212).

The first piece (21) of the cranial component (2) comprises first guiding means (211), preferably dovetail grooves and the second piece (22) of the cranial component (2) comprises second guiding means (221), preferably dovetail protrusions, wherein the first guiding means (211) contribute with the second guiding means (221) to enable detaching the first piece (21) of the cranial component (2) with respect to the second piece (22) of the cranial component (2) when the prosthesis is implanted in the patient.

The cranial component (2) comprises locking means (23), which prevent relative movement between the first piece (21) of the cranial component (2) and the second piece (22) of the cranial component (2) when the prosthesis is implanted in the patient. The locking means (23) are preferably a cover joined by screws to the second piece (22) of the cranial component (2).

The mandibular component (1) comprises a first piece (11) arranged in contact with the first piece (21) of the cranial component (2), wherein the first piece (11) of the mandibular component (1) is exchangeable.

Said first piece (11) of the mandibular component (1) is spherical and attachable to a second piece (12) of the mandibular component (1), which in turn is attachable to the mandible (4) of the patient, preferably attachable to the mandible (4) of the patient by means of screws. The first piece (11) of the mandibular component (1) can be positioned at different heights with respect to the second piece (12) of the mandibular component (1).

The first piece (11) of the mandibular component (1) is housed in the semi-spherical housing (213) of the lower surface (212) of the first piece (21) of the cranial component (2), preferably by means of a snap-fit joint.

The temporomandibular joint replacement system further comprises auxiliary elements (51, 52, 53, 54, 55) configured to carry out implantation of the prosthesis in the patient, preferably being at least one cutting guide (51) of the mandibular component, at least one cutting guide (52) of the cranial component, at least one simple cutting indicator (53) of the cranial component, at least one flanged cutting indicator (54) of the cranial component, at least one cutting indicator (55) of the mandibular component (55), and at least one spacing indicator (56).

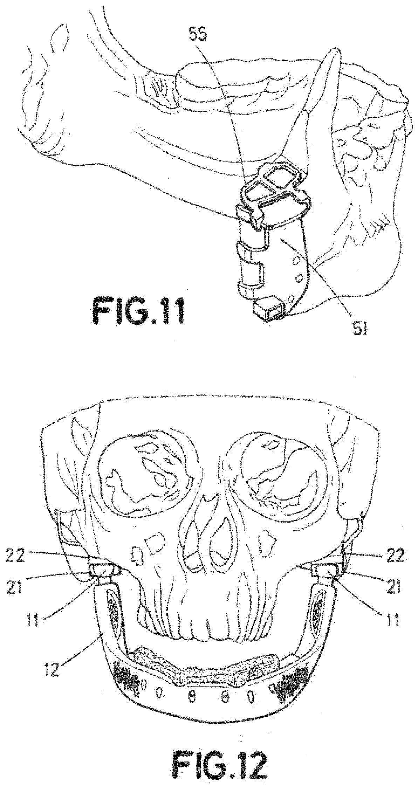

In other exemplary embodiments, when there are serious defects in the mandible and it is necessary to remove the entire mandible, the prosthesis of the temporomandibular joint replacement system comprises:
- a mandibular component (1), and
- a cranial component (2) comprising two first pieces (21) which are arranged in contact with the mandibular component (1),
wherein the first pieces (21) of the cranial component (2) are detachable from the cranial component when the prosthesis is implanted in the patient, as seen in Figure 12.

The first piece (21) of the cranial component (2) is preferably made of ultra-high-molecular-weight-polyethylene (UHMWPE).

The second piece (22) of the cranial component (2) is preferably made of titanium (Ti₆AI₄V).

The mandibular component (1) is preferably made of titanium (Ti₆AI₄V).

The procedure for the implantation of the temporomandibular joint replacement system is described below by way of illustration only.

In general terms, the implantation procedure of the replacement system consists of an intervention wherein the damaged temporomandibular joint is exposed by means of a preauricular or endaural approach combined with a retromandibular or submandibular approach in order to expose the mandibular ramus. Once the damaged area is exposed, the osteotomies or cuts are performed and the recontouring of the surfaces is carried out exactly the same as the planned surfaces.

The design of the prosthesis of the temporomandibular joint replacement system of the present invention is 100% customized to the anatomy of the patient, creating trabecular titanium surfaces for the areas to be filled between the second piece (22) of the cranial component (2) and the cranial component (2) and between a support step of the mandibular component (1) and the second piece (12) of the mandibular component (1).

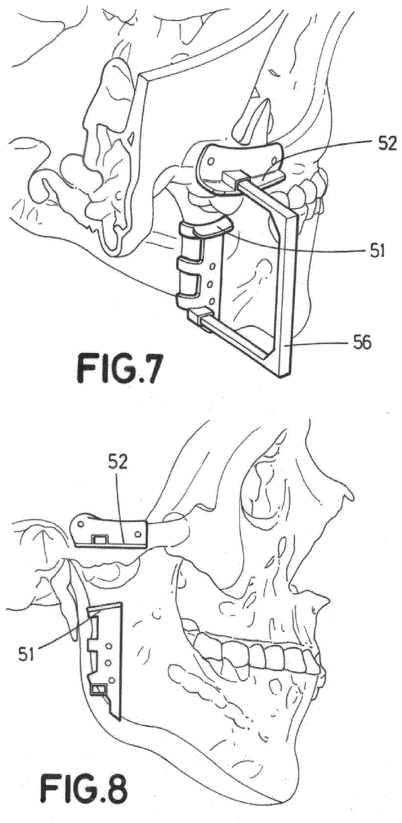

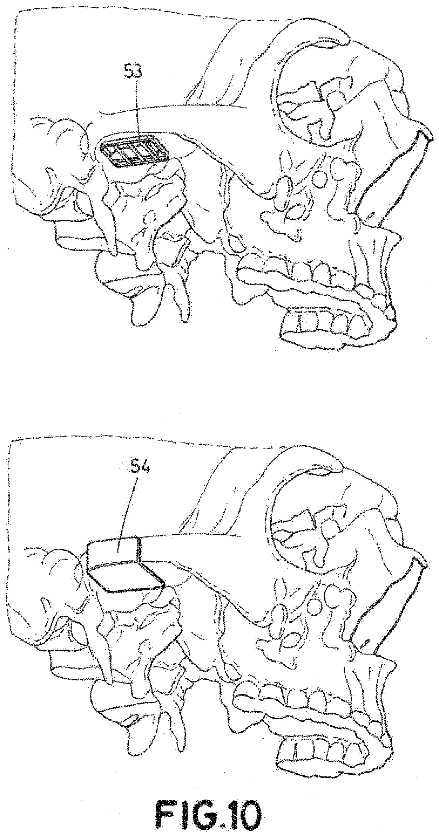

The implantation procedure of the temporomandibular joint replacement system comprises 4 surgical stages:
- Placing the cutting guides (51, 52) and the spacing indicator (56) as shown in Figure 7;
- Performing the planned osteotomies or cuts following the cutting guides (51, 52) intended for said purpose and placed exactly in the right place as a result of the design thereof customized to the anatomy of the patient and the spacing indicator (56) which certifies that said cutting guides are perfectly placed (51, 52).
- Placing the cutting indicators (53, 54, 55) in the mandibular component (1) and in the cranial component (2), as shown in Figures 10 and 11;
- Placing the prosthesis starting with the placement of the second piece (12) of the mandibular component (1) by screwing through holes found in said second piece (12), since said holes coincide with holes on the cutting guide (51) of the mandibular component, placing the second piece (12) of the mandibular component (1) together with the first piece (11) of the mandibular component (1) and then placing the first piece (21) together with the second piece (22) of the cranial component (2), screwing through holes found on said second piece (22) of the cranial component (22), which do not have to coincide with holes in the cutting guide (52) of the cranial component. The placement of the prosthesis can be done inversely, that is, placing the cranial component (2) first and then placing the mandibular component (1).

## Claims

1. A temporomandibular joint replacement system, comprising at least one prosthesis, which in turn comprises:
• a mandibular component (1), and
• a cranial component (2) comprising a first piece (21) which is arranged in contact with the mandibular component (1),
wherein the first piece (21) of the cranial component (2) is detachable from the rest of the cranial component (2) when the prosthesis is implanted in the patient,
wherein the mandibular component (1) comprises:
• a first piece (11) arranged in contact with the first piece (21) of the cranial component (2), wherein the first piece (11) of the mandibular component (1) is exchangeable, and
• a second piece (12), which is attachable to the mandible (4) of the patient, **characterized in that** the first piece (11) of the mandibular component (1) is suitable to be positioned at different heights with respect to the second piece (12) of the mandibular component (1).

2. The temporomandibular joint replacement system according to claim 1, **characterized in that** the cranial component (2) comprises a second piece (22) configured to be anchored to the zygomatic arch (3) of the patient, wherein the first piece (21) of the cranial component (2) is detachable from the second piece (22) of the cranial component (2) when the prosthesis is implanted in the patient.

3. The temporomandibular joint replacement system according to claim 1, **characterized in that** the cranial component (1) comprises guiding means (211, 221) configured to enable detaching the first piece (21) of the cranial component (2) with respect to said cranial component (2) when the prosthesis is implanted in the patient.

4. The temporomandibular joint replacement system according to claims 2 and 3, **characterized in that** the first piece (21) of the cranial component (2) comprises first guiding means (211), preferably dovetail grooves and the second piece (22) of the cranial component (2) comprises second guiding means (221), preferably dovetail protrusions, wherein the first guiding means (211) contribute with the second guiding means (221) to enable detaching the first piece (21) of the cranial component (2) with respect to the second piece (22) of the cranial component (2) when the prosthesis is implanted in the patient.

5. The temporomandibular joint replacement system according to any of the preceding claims, **characterized in that** the cranial component (2) comprises locking means (23), preventing relative movement between the first piece (21) of the cranial component (2) and the cranial component (2) when the prosthesis is implanted in the patient.

6. The temporomandibular joint replacement system according to claims 2 and 5, **characterized in that** the second piece (22) of the cranial component (2) comprises locking means (23), preventing relative movement between the first piece (21) of the cranial component (2) and the second piece (22) of the cranial component (2) when the prosthesis is implanted in the patient.

7. The temporomandibular joint replacement system according to claim 6, **characterized in that** the locking means (23) are preferably a cover joined by screws to the second piece (22) of the cranial component (2).

8. The temporomandibular joint replacement system according to claim 1, **characterized in that** the first piece (11) of the mandibular component (1) is spherical.

9. The temporomandibular joint replacement system according to claim 2, **characterized in that** the second piece (22) of the cranial component (2) is configured to be anchored to the zygomatic arch (3) of the patient by means of a lateral surface (223).

10. The temporomandibular joint replacement system according to claim 9, **characterized in that** the lateral surface (223) of the second piece (22) of the cranial component (2) comprises holes for anchoring by screwing the second piece (22) of the cranial component (2) to the zygomatic arch (3) of the patient.

11. The temporomandibular joint replacement system according to any of the preceding claims, **characterized in that** the first piece (21) of the cranial component (2) comprises a lower surface (212) arranged in contact with the mandibular component (1), wherein said lower surface (212) is preferably cylindrical or frustoconical.

12. The temporomandibular joint replacement system according to claim 11, **characterized in that** the lower surface (212), which is arranged in contact with the mandibular component (1), comprises a semi-spherical housing (213), preferably with a diameter of 8 mm.

13. The temporomandibular joint replacement system according to claim 2, **characterized in that** the first piece (21) of the cranial component (2) comprises an upper surface (214) and the second piece (22) of the cranial component (2) comprises an inner surface (222), wherein the upper surface (212) of the first piece (21) of the cranial component (2) is configured to slide along the inner surface (222) of said lower surface (212).

14. The temporomandibular joint replacement system according to claim 12, **characterized in that** the first piece (11) of the mandibular component (1) is housed in the semi-spherical housing (213) of the lower surface (212) of the first piece (21) of the cranial component (2), preferably by means of a snap-fit joint.

15. The temporomandibular joint replacement system according to any of the preceding claims, **characterized in that** it further comprises auxiliary elements (51, 52, 53, 54, 55) configured to carry out implantation of the prosthesis in the patient, preferably being at least one cutting guide (51, 52) and/or at least one cutting indicator (53, 54, 55) and/or at least one spacing indicator (56).

16. The temporomandibular joint replacement system according to claim 15, **characterized in that** the auxiliary elements (51, 52, 53, 54, 55) configured to carry out implantation of the prosthesis in the patient are at least one cutting guide (51) of the mandibular component, at least one cutting guide (52) of the cranial component, at least one simple cutting indicator (53) of the cranial component, at least one flanged cutting indicator (54) of the cranial component, at least one cutting indicator (55) of the mandibular component (55), and at least one spacing indicator (56).

17. The temporomandibular joint replacement system according to claim 1, wherein the cranial component (2) further comprises an additional first piece (21) arranged in contact with the mandibular component (1) in an opposite side to the mandibular component (1) than a side wherein the first piece (21) is arranged, wherein the additional piece (21) of the cranial component (2) is detachable from the other pieces of the cranial component (2).

18. The temporomandibular joint replacement system according to any of the preceding claims, **characterized in that** the first piece (21) of the cranial component (2) is made of an ultra-high-molecular-weight-polyethylene (UHMWPE).

19. The temporomandibular joint replacement system according to claim 2, **characterized in that** the second piece (22) of the cranial component (2) is made of titanium (Ti₆AI₄V).

20. The temporomandibular joint replacement system according to any of the preceding claims, **characterized in that** the mandibular component (1) is made of titanium (Ti₆AI₄V).

## Patentansprüche

1. Kiefergelenkersatzsystem, umfassend mindestens eine Prothese, die wiederum umfasst:
• eine Unterkieferkomponente (1), und
• eine Schädelkomponente (2), umfassend ein erstes Teil (21), das in Kontakt mit der Unterkieferkomponente (1) angeordnet ist,
wobei das erste Teil (21) der Schädelkomponente (2) vom Rest der Schädelkomponente (2) abnehmbar ist, wenn die Prothese in den Patienten implantiert wird,
wobei die Unterkieferkomponente (1) umfasst:
• ein erstes Teil (11), das in Kontakt mit dem ersten Teil (21) der Schädelkomponente (2) angeordnet ist, wobei das erste Teil (11) der Unterkieferkomponente (1) austauschbar ist, und
• ein zweites Teil (12), das am Unterkiefer (4) des Patienten anbringbar ist, **dadurch gekennzeichnet, dass** das erste Teil (11) der Unterkieferkomponente (1) geeignet ist, in Bezug auf das zweite Teil (12) der Unterkieferkomponente (1) auf unterschiedlichen Höhen positioniert zu werden.

2. Kiefergelenkersatzsystem nach Anspruch 1, **dadurch gekennzeichnet, dass** die Unterkieferkomponente (2) ein zweites Teil (22) umfasst, das konfiguriert ist, um am Jochbogen (3) des Patienten verankert zu werden, wobei das erste Teil (21) der Schädelkomponente (2) von dem zweiten Teil (22) der Schädelkomponente (2) abnehmbar ist, wenn die Prothese in den Patienten implantiert wird.

3. Kiefergelenkersatzsystem nach Anspruch 1, **dadurch gekennzeichnet, dass** die Schädelkomponente (1) Führungsmittel (211, 221) umfasst, die so konfiguriert sind, dass sie das Abnehmen des ersten Teils (21) der Schädelkomponente (2) in Bezug auf die Schädelkomponente (2) ermöglichen, wenn die Prothese in den Patienten implantiert wird.

4. Kiefergelenkersatzsystem nach Anspruch 2 und 3, **dadurch gekennzeichnet, dass** das erste Teil (21) der Schädelkomponente (2) erste Führungsmittel (211), vorzugsweise Schwalbenschwanzrillen umfasst und das zweite Teil (22) der Schädelkomponente (2) zweite Führungsmittel (221), vorzugsweise Schwalbenschwanzvorsprünge umfasst, wobei die ersten Führungsmittel (211) zusammen mit den zweiten Führungsmitteln (221) dazu beitragen, das Abnehmen des ersten Teils (21) der Schädelkomponente (2) in Bezug auf das zweite Teil (22) der Schädelkomponente (2) zu ermöglichen, wenn die Prothese in den Patienten implantiert wird.

5. Kiefergelenkersatzsystem nach einem der vorhergehenden Ansprüche, **dadurch gekennzeichnet, dass** die Schädelkomponente (2) Verriegelungsmittel (23) umfasst, die eine Relativbewegung zwischen dem ersten Teil (21) der Schädelkomponente (2) und der Schädelkomponente (2) verhindern, wenn die Prothese in den Patienten implantiert wird.

6. Kiefergelenkersatzsystem nach Anspruch 2 und 5, **dadurch gekennzeichnet, dass** das zweite Teil (22) der Schädelkomponente (2) Verriegelungsmittel (23) umfasst, die eine Relativbewegung zwischen dem ersten Teil (21) der Schädelkomponente (2) und dem zweiten Teil (22) der Schädelkomponente (2) verhindern, wenn die Prothese in den Patienten implantiert wird.

7. Kiefergelenkersatzsystem nach Anspruch 6, **dadurch gekennzeichnet, dass** die Verriegelungsmittel (23) vorzugsweise eine Abdeckung sind, die durch Schrauben mit dem zweiten Teil (22) der Schädelkomponente (2) verbunden ist.

8. Kiefergelenkersatzsystem nach Anspruch 1, **dadurch gekennzeichnet, dass** das erste Teil (11) der Unterkieferkomponente (1) kugelförmig ist.

9. Kiefergelenkersatzsystem nach Anspruch 2, **dadurch gekennzeichnet, dass** das zweite Teil (22) der Schädelkomponente (2) konfiguriert ist, mittels einer Seitenoberfläche (223) am Jochbogen (3) des Patienten verankert zu werden.

10. Kiefergelenkersatzsystem nach Anspruch 9, **dadurch gekennzeichnet, dass** die Seitenfläche (223) des zweiten Teils (22) der Schädelkomponente (2) Löcher zum Verankern durch Verschrauben des zweiten Teils (22) der Schädelkomponente (2) mit dem Jochbogen (3) des Patienten umfasst.

11. Kiefergelenkersatzsystem nach einem der vorhergehenden Ansprüche, **dadurch gekennzeichnet, dass** das erste Teil (21) der Schädelkomponente (2) eine untere Oberfläche (212) umfasst, die in Kontakt mit der Unterkieferkomponente (1) angeordnet ist, wobei die untere Oberfläche (212) vorzugsweise zylindrisch oder kegelstumpfförmig ist.

12. Kiefergelenkersatzsystem nach Anspruch 11, **dadurch gekennzeichnet, dass** die untere Oberfläche (212), die in Kontakt mit der Unterkieferkomponente (1) angeordnet ist, ein halbkugelförmiges Gehäuse (213) vorzugsweise mit einem Durchmesser von 8 mm umfasst.

13. Kiefergelenkersatzsystem nach Anspruch 2, **dadurch gekennzeichnet, dass** das erste Teil (21) der Schädelkomponente (2) eine obere Oberfläche (214) umfasst und das zweite Teil (22) der Schädelkomponente (2) eine innere Oberfläche (222) umfasst, wobei die obere Oberfläche (212) des ersten Teils (21) der Schädelkomponente (2) konfiguriert ist, um entlang der inneren Oberfläche (222) der unteren Oberfläche (212) zu gleiten.

14. Kiefergelenkersatzsystem nach Anspruch 12, **dadurch gekennzeichnet, dass** das erste Teil (11) der Unterkieferkomponente (1) in dem halbkugelförmigen Gehäuse (213) der unteren Oberfläche (212) des ersten Teils (21) der Schädelkomponente (2) vorzugsweise mittels einer Schnappverbindung aufgenommen ist.

15. Kiefergelenkersatzsystem nach einem der vorhergehenden Ansprüche, **dadurch gekennzeichnet, dass** es ferner Hilfselemente (51, 52, 53, 54, 55) umfasst, die konfiguriert sind, die Implantation der Prothese in den Patienten durchzuführen, die vorzugsweise mindestens eine Schnittführung (51, 52) und/oder mindestens ein Schnittindikator (53, 54, 55) und/oder mindestens einen Abstandsindikator (56) sind.

16. Kiefergelenkersatzsystem nach Anspruch 15, **dadurch gekennzeichnet, dass** die Hilfselemente (51, 52, 53, 54, 55), die konfiguriert sind, die Implantation der Prothese in den Patienten durchzuführen, mindestens eine Schnittführung (51) der Unterkieferkomponente, mindestens eine Schnittführung (52) der Schädelkomponente, mindestens ein einfacher Schnittindikator (53) der Schädelkomponente, mindestens ein Flanschschnittindikator (54) der Schädelkomponente, mindestens einen Schnittindikator (55) der Unterkieferkomponente (55) und mindestens einen Abstandsindikator (56) sind.

17. Kiefergelenkersatzsystem nach Anspruch 1, wobei die Schädelkomponente (2) ferner ein zusätzliches erstes Teil (21) umfasst, das in Kontakt mit der Unterkieferkomponente (1) auf einer gegenüberliegenden Seite der Unterkieferkomponente (1) angeordnet ist als eine Seite, auf der das erste Teil (21) angeordnet ist, wobei das zusätzliche Teil (21) der Schädelkomponente (2) von den anderen Teilen der Schädelkomponente (2) abnehmbar ist.

18. Kiefergelenkersatzsystem nach einem der vorhergehenden Ansprüche, **dadurch gekennzeichnet, dass** das erste Teil (21) der Schädelkomponente (2) aus einem ultrahochmolekularen Polyethylen (UHMWPE) gefertigt ist.

19. Kiefergelenkersatzsystem nach Anspruch 2, **dadurch gekennzeichnet, dass** das zweite Teil (22) der Schädelkomponente (2) aus Titan (Ti₆AI₄V) gefertigt ist.

20. Kiefergelenkersatzsystem nach einem der vorhergehenden Ansprüche, **dadurch gekennzeichnet, dass** die Unterkieferkomponente (1) aus Titan (Ti₆AI₄V) gefertigt ist.

## Revendications

1. Système de remplacement de l'articulation temporo-mandibulaire, comprenant au moins une prothèse, qui comprend à son tour :
• un composant mandibulaire (1), et
• un composant crânien (2) comprenant une première pièce (21) qui est agencée en contact avec le composant mandibulaire (1),
dans lequel la première pièce (21) du composant crânien (2) est détachable du reste du composant crânien (2) lorsque la prothèse est implantée dans le patient, dans lequel le composant mandibulaire (1) comprend :
• une première pièce (11) agencée en contact avec la première pièce (21) du composant crânien (2), dans lequel la première pièce (11) du composant mandibulaire (1) est échangeable, et
• une seconde pièce (12), qui peut être fixée à la mandibule (4) du patient, **caractérisé en ce que** la première pièce (11) du composant mandibulaire (1) est appropriée pour être positionnée à des hauteurs différentes par rapport à la seconde pièce (12) du composant mandibulaire (1).

2. Système de remplacement de l'articulation temporo-mandibulaire selon la revendication 1, **caractérisé en ce que** le composant crânien (2) comprend une seconde pièce (22) configurée pour être ancrée à l'arcade zygomatique (3) du patient, dans lequel la première pièce (21) du composant crânien (2) est détachable de la seconde pièce (22) du composant crânien (2) lorsque la prothèse est implantée dans le patient.

3. Système de remplacement de l'articulation temporo-mandibulaire selon la revendication 1, **caractérisé en ce que** le composant crânien (1) comprend des moyens de guidage (211,221) configurés pour permettre de détacher la première pièce (21) du composant crânien (2) par rapport audit composant crânien (2) lorsque la prothèse est implantée dans le patient.

4. Système de remplacement de l'articulation temporo-mandibulaire selon les revendications 2 et 3, **caractérisé en ce que** la première pièce (21) du composant crânien (2) comprend des premiers moyens de guidage (211), de préférence des rainures en queue d'aronde et la seconde pièce (22) du composant crânien (2) comprend des seconds moyens de guidage (221), de préférence des saillies en queue d'aronde, dans lequel les premiers moyens de guidage (211) contribuent avec les seconds moyens de guidage (221) pour permettre le détachement de la première pièce (21) du composant crânien (2) par rapport à la seconde pièce (22) du composant crânien (2) lorsque la prothèse est implantée dans le patient.

5. Système de remplacement de l'articulation temporo-mandibulaire selon l'une quelconque des revendications précédentes, **caractérisé en ce que** le composant crânien (2) comprend des moyens de blocage (23), empêchant un déplacement relatif entre la première pièce (21) du composant crânien (2) et le composant crânien (2) lorsque la prothèse est implantée dans le patient.

6. Système de remplacement de l'articulation temporo-mandibulaire selon les revendications 2 et 5, **caractérisé en ce que** la seconde pièce (22) du composant crânien (2) comprend des moyens de blocage (23), empêchant un déplacement relatif entre la première pièce (21) du composant crânien (2) et la seconde pièce (22) du composant crânien (2) lorsque la prothèse est implantée dans le patient.

7. Système de remplacement de l'articulation temporo-mandibulaire selon la revendication 6, **caractérisé en ce que** les moyens de blocage (23) sont de préférence un couvercle solidarisé par des vis à la seconde pièce (22) du composant crânien (2).

8. Système de remplacement de l'articulation temporo-mandibulaire selon la revendication 1, **caractérisé en ce que** la première pièce (11) du composant mandibulaire (1) est sphérique.

9. Système de remplacement de l'articulation temporo-mandibulaire selon la revendication 2, **caractérisé en ce que** la seconde pièce (22) du composant crânien (2) est configurée pour être ancrée à l'arcade zygomatique (3) du patient au moyen d'une surface latérale (223).

10. Système de remplacement de l'articulation temporo-mandibulaire selon la revendication 9, **caractérisé en ce que** la surface latérale (223) de la seconde pièce (22) de l'élément crânien (2) comprend des trous pour ancrer par vissage la seconde pièce (22) de l'élément crânien (2) à l'arc zygomatique (3) du patient.

11. Système de remplacement de l'articulation temporo-mandibulaire selon l'une quelconque des revendications précédentes, **caractérisé en ce que** la première pièce (21) du composant crânien (2) comprend une surface inférieure (212) agencée en contact avec le composant mandibulaire (1), dans lequel ladite surface inférieure (212) est de préférence cylindrique ou tronconique.

12. Système de remplacement de l'articulation temporo-mandibulaire selon la revendication 11, **caractérisé en ce que** la surface inférieure (212), qui est agencée en contact avec le composant mandibulaire (1), comprend un logement semi-sphérique (213), de préférence avec un diamètre de 8 mm.

13. Système de remplacement de l'articulation temporo-mandibulaire selon la revendication 2, **caractérisé en ce que** la première pièce (21) du composant crânien (2) comprend une surface supérieure (214) et la seconde pièce (22) du composant crânien (2) comprend une surface interne (222), dans lequel la surface supérieure (212) de la première pièce (21) du composant crânien (2) est configurée pour coulisser le long de la surface interne (222) de ladite surface inférieure (212).

14. Système de remplacement de l'articulation temporo-mandibulaire selon la revendication 12, **caractérisé en ce que** la première pièce (11) de l'élément mandibulaire (1) est logée dans le logement semi-sphérique (213) de la surface inférieure (212) de la première pièce (21) de l'élément crânien (2), de préférence au moyen d'un joint à encliquetage.

15. Système de remplacement de l'articulation temporo-mandibulaire selon l'une quelconque des revendications précédentes, **caractérisé en ce qu'**il comprend en outre des éléments auxiliaires (51, 52, 53, 54, 55) configurés pour réaliser l'implantation de la prothèse dans le patient, étant de préférence au moins un guide de coupe (51, 52) et/ou au moins un indicateur de coupe (53, 54, 55) et/ou au moins un indicateur d'espacement (56).

16. Système de remplacement de l'articulation temporo-mandibulaire selon la revendication 15, **caractérisé en ce que** les éléments auxiliaires (51, 52, 53, 54, 55) configurés pour réaliser l'implantation de la prothèse dans le patient sont au moins un guide de coupe (51) du composant mandibulaire, au moins un guide de coupe (52) du composant crânien, au moins un indicateur de coupe simple (53) du composant crânien, au moins un indicateur de coupe à onglet (54) du composant crânien, au moins un indicateur de coupe (55) du composant mandibulaire (55), et au moins un indicateur d'espacement (56).

17. Système de remplacement de l'articulation temporo-mandibulaire selon la revendication 1, dans lequel le composant crânien (2) comprend en outre une première pièce supplémentaire (21) agencée en contact avec le composant mandibulaire (1) dans un côté opposé au composant mandibulaire (1) qu'un côté dans lequel la première pièce (21) est agencée, dans lequel la pièce supplémentaire (21) du composant crânien (2) est détachable des autres pièces du composant crânien (2).

18. Système de remplacement de l'articulation temporo-mandibulaire selon l'une quelconque des revendications précédentes, **caractérisé en ce que** la première pièce (21) du composant crânien (2) est constituée d'un polyéthylène à ultra-haut poids moléculaire (UHMWPE).

19. Système de remplacement de l'articulation temporo-mandibulaire selon la revendication 2, **caractérisé en ce que** la seconde pièce (22) du composant crânien (2) est constituée de titane (Ti₆AI₄V).

20. Système de remplacement de l'articulation temporo-mandibulaire selon l'une quelconque des revendications précédentes, **caractérisé en ce que** le composant mandibulaire (1) est constitué de titane (Ti₆AI₄V).
